# EUROPÄISCHE PATENTSCHRIFT

(11) **EP 0 826 008 B1**
(45) Veröffentlichungstag und Bekanntmachung des Hinweises auf die Patenterteilung: **14.07.1999**
(21) Anmeldenummer: 96916036.5
(22) Anmeldetag: 10.05.1996
(51) Int. Cl.: C08F 8/12, C09D 129/04, C09J 129/04, C08K 5/098

(54) **POLYVINYLESTER-DISPERSION MIT METALLSALZ-HYDROSOLEN ALS SAATGRUNDLAGE**
POLYVINYL ESTER DISPERSION WITH METAL SALT HYDROSOLS AS PRECIPITATION BASIS
DISPERSION D'ESTER POLYVINYLIQUE COMPRENANT DES HYDROSOLS DE SELS METALLIQUES POUR BASE DE PRECIPITATION

(30) Priorität: 15.05.1995 DE 19517777
(43) Veröffentlichungstag der Anmeldung: 04.03.1998
(73) Patentinhaber: Clariant GmbH, 65929 Frankfurt am Main (DE)
(72) Erfinder: JAKOB, Martin, D-65779 Kelkheim (DE); HENNEMANN, Heinz, D-65558 Oberneisen (DE); MATZ, Volker, D-60529 Frankfurt (DE)
(86) Internationale Anmeldenummer: EP9601999
(87) Internationale Veröffentlichungsnummer: WO9636648

(56) Entgegenhaltungen:
- EP-A- 0 069 301
- EP-A- 0 433 957
- US-A- 2 455 937
- US-A- 3 941 730

## Beschreibung

Die Erfindung betrifft oberflächenaktive Substanzen enthaltende Hydrosole von Metallkomplexsalzen sowie ein spezielles Verfahren der radikalischen Emulsionspolymerisation von Vinylestern in Gegenwart dieser Hydrosole als Saatgrundlage. Gegenstand der Erfindung ist weiterhin die Verwendung derart hergestellter Polyvinylester-Dispersionen als Klebstoff für poröse oder semiporöse Substrate.

Wäßrige Dispersionen von Polyvinylestern, insbesondere die von Polyvinylacetat, werden als sogenannte Weißleime zum Kleben von Holz und anderen porösen Substraten verwendet. Solche Klebstoffe sind in der Literatur beschrieben worden, beispielsweise in Handbook of Adhesives, 3. Auflage, Kapitel 21, Van Nostrand Reinhold, New York, 1990 und in Wood Adhesives - Chemistry and Technology, Band 1, Kapitel 7, Marcel Dekker, New York, 1983.

Die Herstellung dieser Klebstoffe erfolgt gewöhnlich durch radikalische Emulsionspolymerisation der Vinylester in Gegenwart von polymeren Stabilisatoren, sogenannten Schutzkolloiden, insbesondere Polyvinylalkohol. Eine Übersicht über dieses in der Fachliteratur umfangreich behandelte Verfahren findet sich beispielsweise in Wood Adhesives - Chemistry and Technology, Band 2, Kapitel 2, Marcel Dekker, New York, 1989.

Gewöhnlich liegen nach der Emulsionspolymerisation von Vinylestern, insbesondere Vinylacetat, in Gegenwart von Polyvinylalkohol Dispersionen vor, die relativ hochviskos sind. Außerdem findet man im Vergleich zu Dispersionen, die ausschließlich mit Hilfe von reinen Emulgatoren stabilisiert sind, breitere Partikelgrößenverteilungen vor. Abhängig vom Polymerisationsverfahren, der Art der Initiatoren, der Art der Stabilisatoren und weiterer Einflußgrößen werden gewöhnlich darüber hinaus auch Partikelagglomerationen beobachtet. Dieses Phänomen bedingt vor allem eine weitere Erhöhung der Viskosität der Dispersionen bis hin zu pastöser Konsistenz und einer Beeinträchtigung des Fließens. Dies verursacht auch einen Verlust der Scherstabilität und verschlechtert allgemein die reproduzierbare Herstellung von Dispersionen in engen Viskositätsgrenzen.

Eine Untergruppe der Polyvinylester-Dispersionen bilden solche mit erhöhter Wasserresistenz ihrer Klebverbindungen. In diesen wird häufig die Hydrophilie des als Schutzkolloid verwendeten Polyvinylalkohols durch Copolymerisation vernetzend wirkender Vinylverbindungen wie N-Methylol(meth)acrylamid vermindert.

Ein solcher Weg wird beispielsweise in den Schriften US-A 3,301,809, DE-C 26 20 738 und DE-A 39 42 628 vorgeschlagen. In Kombination mit sauren Härtern wie wäßrigen Mineralsäuren oder aciden Metallsalzen, beispielsweise wäßrigen Lösungen von Aluminiumchlorid, werden wasserresistente Klebstoffe erhalten.

Ein bekannter Nachteil des Verfahrens besteht darin, daß durch die Einpolymerisation steigender Mengen des Vernetzungsmittels N-Methylol(meth)acrylamid eine weitere drastische Zunahme der Viskosität beobachtet wird. Darüberhinaus tendieren die Dispersionen beim Lagern, insbesondere in der sauer katalysierten Form, zu Viskositäts- und Strukturaufbau. Die Topfzeit der sauer katalysierten Leimansätze ist daher gewöhnlich gering.

In den Schriften DE-C 26 20 738 und DE-A 39 42 628 wird ein zweistufiges Polymerisationsverfahren beschrieben, bei dem ein Monomergemisch partiell in der wäßrigen Flotte vorgelegt und partiell dosiert wird. Bei den Monomeren handelt es sich hauptsächlich um Vinylester der Essigsäure, Pivalinsäure und ®Versaticsäuren (α,α-Dialkylcarbonsäuren, Shell-Chemie) sowie N-Methylolacrylamid. In Abhängigkeit von der Art der Vinylester sowie der Art der nachträglich zugesetzten sauren Katalysatoren werden Leimzubereitungen erhalten, die Topfzeiten von mindestens 4 Wochen aufweisen.

In US-A 4 085 074 werden flüchtige Stickstoffbasen wie Ammoniak, Mono-, Di- oder Trialkyl(ol)amine, insbesondere Triethanolamin als Vernetzungsinhibitoren für die sauren Metallsalze wie Aluminiumchlorid vorgeschlagen. Die Basen werden den Leimansätzen nachträglich zugegeben. Die Lagerstabilität der so modifizierten Zubereitungen überschreitet 3 Monate, jedoch liegen ihre Viskositäten vor Beginn der Lagerung mit < 1,5 Pa·s deutlich unterhalb den heutigen Marktforderungen für solche Systeme von mindestens 7 Pa·s. Weiterhin neigen Dispersionen, die Stickstoffbasen enthalten, beim Lagern häufig zu Vergilbung.

Beide vorgenannten Lösungsansätze lösen zudem nicht das Problem der drastischen Zunahme der Viskositäten von unter Verwendung steigender Mengen N-Methylol(meth)acrylamid hergestellten Polyvinylesterdispersionen.

Bei dem in DE-C 26 20 738 und DE-A 39 42 628 angewandten Verfahren handelt es sich offenbar um eine Pfropfreaktion auf einer während der Anfangsphase der Polymerisation erzeugten Partikelsaat, bestehend aus einem bereits vorvernetzten und mit Polyvinylalkohol stabilisiertem Polymerisat.

Das Prinzip einer Saatpolymerisation herkömmlicher Vorgehensweise besteht grundsätzlich darin, daß vor der Emulsionspolymerisation in der wäßrigen Flotte eine gewisse Menge einer bereits in einem zuvor durchgeführten Verfahrensschritt hergestellten Dispersion vorgelegt wird. Die Polymerisation wird auf diese Weise in Gegenwart bereits schon vorhandener Partikel mit kontrollierten Mengen an Emulgator und Initiator durchgeführt, so daß spezielle Partikelgrößenverteilungen ermöglicht werden (siehe J. W. Vanderhoff, Chem. Engin. Sc. 48, 203 (1993)). Ein Nachteil einer solchen Vorgehensweise ist jedoch, daß zur Herstellung einer solchen Dispersion zwei Polymerisationsschritte erforderlich sind.

In der Literatur finden sich weiterhin zahlreiche Varianten von Saatpolymerisationen, bei denen statt eines Polymerisat-Saatlatex eine anorganische Partikelsaat wie beispielsweise ein Pigment vor der Emulsionspolymerisation vorgelegt wird. Beispiele hierfür sind feinverteiltes Calciumcarbonat, Titandioxid oder Silicagel-Partikel, die während der Polymerisation durch das aufwachsende Polymere verkapselt werden. Eine Literaturübersicht geben A. Revillon et al. in Double Liaison 431432, 285 (1991).

EP-B 0 337 672 beschreibt die Emulsionspolymerisation von Vinylestern in Gegenwart eines hohen Anteils an Titandioxid-Pigmentpartikeln unter Verwendung eines Redoxinitiatorsystems überwiegend in Gegenwart von polymeren Stabilisatoren wie Hydroxyethylcellulose und Polyacrylsäure wie auch eines anionischen Emulgators mit dem Ergebnis, daß die Pigmentteilchen nicht verkapselt werden, sondern in Form diskreter Partikel, von Polymerteilchen umgeben, in Form von knotenähnlichen Konturen vorliegen. Die Dispersionen finden Anwendung als Oberflächenbeschichtungsmaterial mit verbesserter Opaztät und einer höheren High-Shear-Viskosität.

Aus der Literatur (A. Clearfield, Rev. Pure and Appl. Chem. 14, 91 (1964)) ist bekannt, daß mit Zirkonium (IV) und zweizähnigen Sauerstoff-Liganden stabile Komplexverbindungen erhältlich sind, die aus den sauren wäßrigen Lösungen von Salzen des Zirkoniums (IV) nach Zugabe niedermolekularer α-Hydroxycarbonsäuren, beispielsweise Glykolsäure, in gelatinöser, amorpher Form ausfallen. Hierbei handelt es sich auch um Chelatkomplexe, in denen die α-Hydroxycarbonsäure als zweizähniger Sauerstoff-Ligand auftritt.

Der vorliegenden Erfindung lag die Aufgabe zugrunde, ein Verfahren der radikalischen Emulsionspolymerisation zur Herstellung von überwiegend mit Schutzkolloiden wie Polyvinylalkohol stabilisierten Polyvinylester-Dispersionen zu entwickeln, das über eine verringerte Tendenz zur Partikelagglomeration eine verbesserte rheologische Kontrolle der Endprodukte mit sich bringt und auch bei der Einpolymerisation größerer Mengen an vernetzend wirkenden Comonomeren wie N-Methylol(meth)acrylamid niedrigere Latexviskositäten und nach Zugabe saurer Vernetzungskatalysatoren, beispielsweise wäßrigen Lösungen von Aluminiumchlorid, den so katalysierten Leimansätzen eine verlängerte Lagerstabilität verleiht.

Es wurde nun überraschend gefunden, daß beide Aspekte dieser Aufgabe durch ein neuartiges Verfahren der Emulsionspolymerisation gelöst werden können, das dadurch gekennzeichnet ist, daß eine Saatpolymerisation durchgeführt wird, indem als Teilchensaat in der wäßrigen Polymerisationsflotte vor der Polymerisation ein kolloiddisperses Komplexsalz einer organischen Säure mit einem Metall der 4. Nebengruppe des Periodensystems, insbesondere Zirkonium, vorgelegt wird, das mit mindestens einer oberflächenaktiven Substanz, insbesondere Polyvinylalkohol, stabilisiert wird.

Ein Gegenstand der Erfindung ist ein eine oberflächenaktive Substanz, vorzugsweise ein überwiegend polymeres Stabilisierungsmittel, insbesondere Polyvinylalkohol, enthaltendes Hydrosol eines Komplexsalzes eines Metalls der 4. Nebengruppe des Periodensystems, insbesondere Zirkonium, mit einer organischen α-Hydroxy- und/oder α-Oxosäure.

Die Teilchengrößen der Hydrosole, die als Teilchensaat für eine nachfolgende Emulsionspolymerisation geeignet sind, bewegen sich gewöhnlich in den Größenordnungen von 200 nm bis 5 µm. Unter dem Elektronenmikroskop erweisen sich die Hydrosolpartikel ihrerseits als Aggregate noch kleinerer, etwa 20 nm großer Primärpartikel.

Das bevorzugt in den erfindungsgemäßen Hydrosolen enthaltene Stabilisierungsmittel ist Polyvinylalkohol vom Hydrolysegrad von 60 bis 100 Mol-%, insbesondere 70 bis 98 Mol-%, dessen 4 Gew.-%igen wäßrigen Lösungen vorzugsweise Viskositäten von 2 bis 70 mPa·s bei 20 °C besitzen. Vorzugsweise geeigneter Polyvinylalkohol ist derselbe, der später in der wäßrigen Polymerisationsflotte zur Stabilisierung der Polyvinylester-Dispersion verwendet wird. Es können daher auch Gemische verschiedener Produkte mit unterschiedlichen Hydrolysegraden und Molekulargewichten eingesetzt werden.

Werden in den kolloiddispersen Komplexsalzen andere polymere Stabilisierungsmittel verwendet oder mitverwendet, sind Schutzkolloide geeignet, die keine freien Carboxylgruppen oder andere mit Zirkoniumionen stark komplexbildende funktionelle Gruppen aufweisen. Beispielhaft genannt seien hierzu Hydroxyethylcellulose, Stärke, Stärkederivate, Dextrin und Polyvinylpyrrolidon.

Die Mitverwendung kleiner Mengen an anionischen, nichtionischen oder kationischen Emulgatoren ist zulässig. Vorzugsweise werden nichtionische Emulgatoren wie beispielsweise ethoxylierte Fettalkohole mitverwendet. Deren Menge wird vorzugsweise so bemessen, daß ihre Gesamtmenge, bezogen auf die Masse der Monomeren, in der späteren Polymerisation 0 bis 3 Gew.-%, vorzugsweise 0,005 bis 2 Gew.-%, beträgt.

Die Masse der zur Stabilisierung der Komplexverbindung verwendeten oberflächenaktiven Substanzen wird vorzugsweise so bemessen, daß pro mmol Zirkonium 0,5 bis 100 g, insbesondere 1 bis 20 g, besonders bevorzugt 2 bis 5 g oberflächenaktive Substanzen verwendet werden.

Die erfindungsgemäßen Hydrosole enthalten mindestens ein Element der 4. Nebengruppe des Periodensystems, insbesondere Zirkonium. Es können auch Gemische eines Elements der 4. Nebengruppe mit weiteren Elementen dieser Gruppe oder anderen metallischen Elementen vorliegen.

Bei in den erfindungsgemäßen Hydrosolen enthaltenen organischen α-Hydroxy- und/oder α-Oxosäuren handelt es sich vorzugsweise um solche, die im sauren Medium mit wasserlöslichen Zr(IV)-Salzen unlösliche Komplexverbindungen bilden. Bevorzugt handelt es sich um eine mono- oder polyfunktionelle Carbonsäure, die in α-Stellung zu mindestens einer Carbonylgruppe eine Carbonylgruppe aufweist, die in wäßriger Phase im Gleichgewicht in vollständig oder partiell hydratisierter Form vorliegt. Erfindungsgemäß insbesondere geeignete Säuren, die α-ständig zu mindestens einer Carboxylgruppe eine Hydroxylgruppe tragen, sind Glykolsäure, Acrylamidoglykolsäure, Milchsäure, Zitronensäure, Mandelsäure und Weinsäure. Weiterhin vorzugsweise geeignete Verbindungen, die zusätzlich zu einer Carboxylgruppe eine zu ihr α-ständige Carbonylgruppe und damit im wäßrigen Gleichgewicht in hydratisierter Form zwei zur Carboxylgruppe α-ständige geminale Hydroxylgruppen aufweisen, sind beispielsweise die Glyoxylsäure und Brenztraubensäure. Insbesondere die Glyoxylsäure ist zur Herstellung eines erfindungsgemäßen Hydrosols als Teilchensaat für die spätere Emulsionspolymerisation besonders geeignet und bevorzugt.

Das molare Verhältnis zwischen den verwendeten organischen α-Hydroxy- und/oder α-Oxocarbonsäuren und den wäßrigen Zr(lV)-Salzen, ist für die später bei der Emulsionspolymerisation beobachteten Effekte auf die Partikelgrößenverteilung der Dispersionen, von großer Bedeutung. Das molare Verhältnis von Carbonsäure zu Zirkonium soll dabei vorzugsweise 0,001 bis 4, insbesondere 0,05 bis 2, besonders bevorzugt 0,08 bis 1,2 betragen.

Ein Gegenstand der vorliegenden Erfindung ist auch ein Verfahren zur Herstellung eines Hydrosols eines Komplexsalzes eines Metalls der 4. Nebengruppe des Periodensystems, insbesondere Zirkonium, mit einer organischen α-Hydroxy- und/oder α-Oxosäure durch Fällung aus homogener wäßriger Lösung eines wasserlöslichen Salzes dieses Metalls durch Zugabe einer organischen α-Hydroxy- und/oder α-Oxosäure oder einer wäßrigen oder organischen Lösung dieser organischen Säure in Gegenwart einer oberflächenaktiven Substanz, vorzugsweise eines überwiegend polymeren Stabilisierungsmittels, insbesondere Polyvinylalkohol.

Die Herstellung der besonders bevorzugten mit Polyvinylalkohol stabilisierten Zr(lV)-Komplexsalz-Hydrosole, die als Teilchensaat für eine nachfolgende Emulsionspolymerisation geeignet sind, geschieht beispielsweise in einfacher Weise dadurch, daß zunächst eine wäßrige Lösung von Polyvinylalkohol definierter Konzentration hergestellt wird. Die Konzentration der Polyvinylalkohol-Lösung beträgt während der Herstellung des Hydrosols vorzugsweise 1 bis 20 Gew.-%. Insbesondere ist die Konzentration der Lösung dieselbe, die später die verwendete Polymerisationsflotte aufweist. In dieser Lösung wird unter Rühren die α-Hydroxycarbonsäure oder die α-Oxocarbonsäure gelöst und eine wäßrige Lösung eines sauren wasserlöslichen Zr(IV)-Salzes zugegeben. Bei den verwendeten sauren wasserlöslichen Zr(lV)-Salzen handelt es sich beispielsweise um wäßrige Lösungen von Zirkoniumoxychlorid, Zirkoniumnitrat, Zirkoniumorthosulfat und Zirkoniumacetat. Wegen der im Vergleich zu den stark sauren Salzen des Zirkoniumoxychlorids, Zirkoniumnitrats und Zirkoniumorthosulfats schwächeren Acidität des Zirkoniumacetats ist dieses besonders bevorzugt, da nach Zugabe dieser Lösung bereits ein für die spätere Emulsionspolymerisation geeigneter pH-Wert zwischen 3 und 5 vorliegt. Selbstverständlich kann der pH-Wert des Hydrosols auch nach dessen Herstellung durch geeignete Neutralisationsmittel wie verdünnte Natronlauge nachträglich auf die geforderten Werte angehoben werden. Im Falle des Zirkoniumacetats resultiert, bedingt durch die freigewordene Essigsäure, auf diese Weise ein Puffersystem, das rein durch Änderungen des pH-Werts bedingte Viskositätserhöhungen beim Einsatz in Dispersionen unterdrückt. Die mit anderen Salzen hergestellten Hydrosole können durch Zugabe geeigneter Puffersysteme modifiziert werden. Nach Ablauf einer Zeitspanne, die von der Reaktionstemperatur abhängig ist, bildet sich das Hydrosol, erkennbar an einer beginnenden milchig-trüben Konsistenz der Lösung. Die geeignete Reaktionstemperatur beträgt 20 bis 90 °C, vorzugsweise 30 bis 80 °C, insbesondere 40 bis 60 °C. Gewöhnlich bildet sich das Hydrosol in dieser Temperaturspanne innerhalb von Minuten. Es kann dann zu einer Polymerisationsflotte gegeben werden oder vorzugsweise auch selbst im Eintopfverfahren als Polymerisationsflotte für die nachfolgende Emulsionspolymerisation verwendet werden.

Durch Art und Molekulargewicht des verwendeten Stabilisierungsmittels, durch dessen Konzentration in der Lösung und damit durch das Massenverhältnis des Stabilisierungsmittels, bezogen auf die Komplexverbindung, sowie durch die Rührgeschwindigkeit während der Reaktion lassen sich die Partikelgrößen der Hydrosole variieren. Dabei ist insbesondere bei Einsatz von Polyvinylalkohol durch Erhöhung des Molekulargewichts und der Konzentration in der Lösung sowie durch Erhöhung der Rührgeschwindigkeit eine deutliche Verkleinerung der Partikelgrößen der Hydrosole zu beobachten.

Gegenstand der Erfindung ist auch eine Polyvinylester-Dispersion, erhältlich durch Emulsionspolymerisation wenigstens eines Vinylester-Monomeren und gegebenenfalls weiterer copolymerisationsfähiger Monomerer, in Gegenwart eines eine oberflächenaktive Substanz, vorzugsweise ein überwiegend polymeres Stabilisierungsmittel, insbesondere Polyvinylalkohol, enthaltenden Hydrosols eines Komplexsalzes eines Metalls der 4. Nebengruppe des Periodensystems, insbesondere Zirkonium, mit einer organischen α-Hydroxy- und/oder α-Oxosäure.

Die Monomerbasis für die erfindungsgemäße homo- oder copolymere Polyvinylester-Dispersion können Vinylformiat, Vinylacetat, Vinylpropionat, Vinylisobutyrat, Vinylpivalat, Vinyl-2-ethylhexanoat, Vinylester von gesättigten α-verzweigten Monocarbonsäuren mit 9 bis 10 Kohlenstoffatomen im Säurerest, Vinylester von längerkettigen gesättigten oder ungesättigten Fettsäuren, beispielsweise Vinyllaurat, Vinylstearat, sowie Vinylester der Benzoesäure und substituierter Derivate der Benzoesäure wie Vinyl-p-tert.-butylbenzoat bilden. Unter diesen ist Vinylacetat besonders bevorzugt. Die genannten Vinylester können im Polyvinylester auch nebeneinander vorliegen. Der Anteil aller vorgenannten Vinylester im Polymerisat beträgt vorzugsweise mindestens 50 Gew.-%. Der Anteil an Vinylacetat am Gesamtanteil der Vinylester in der copolymeren Polyvinylester-Dispersion beträgt vorzugsweise mindestens 50 Gew.-%, insbesondere mindestens 75 Gew.-%.

Weitere ethylenisch ungesättigte Monomere, die mit den Vinylestern copolymerisiert werden können, sind α,β-ungesättigte Säuren, beispielsweise Acrylsäure, Methacrylsäure, sowie deren Ester mit primären und sekundären gesättigten einwertigen Alkoholen mit 1 bis 18 Kohlenstoffatomen, beispielsweise Methanol, Ethanol, Propanol, Butanol, 2-Ethylhexylalkohol, cycloaliphatischen Alkoholen sowie längerkettigen Fettalkoholen. Weiterhin können α,β-ungesättigte Dicarbonsäuren, beispielsweise Maleinsäure, Fumarsäure, Itaconsäure oder Citraconsäure, sowie deren Mono- oder Diester mit gesättigten einwertigen aliphatischen Alkoholen mit 1 bis 18 Kohlenstoffatomen mitverwendet werden. Der Anteil dieser Comonomere an der Gesamtmonomerenmenge beträgt bis zu 25 Gew.-%, vorzugsweise 0,1 bis 15 Gew.-%.

Geeignete Comonomere sind weiterhin ethylenisch ungesättigte Kohlenwasserstoffe, wie Ethylen oder α-Olefine mit 3-18 Kohlenstoffatomen, beispielsweise Propylen, Butylen, ferner Styrol, Vinyltoluol, Vinylxylol sowie halogenierte ungesättigte aliphatische Kohlenwasserstoffe, beispielsweise Vinylchlorid, Vinylfluorid, Vinylidenchlorid, Vinylidenfluorid. Der Anteil dieser Comonomere an der Gesamtmonomerenmenge beträgt bis zu 50 Gew.-%, vorzugsweise 1 bis 25 Gew.-%.

Weiterhin können während der Polymerisation vernetzende mehrfach ethylenisch ungesättigte Monomere beispielsweise Diallylphthalat, Diallylmaleinat, Triallylcyanurat, Tetraallyloxyethan, Divinylbenzol, Butandiol-1,4-dimethacrylat, Triethylenglykoldimethacrylat, Divinyladipat, Allyl(meth)-acrylat, Vinylcrotonat, Methylenbisacrylamid, Hexandioldiacrylat, Pentaerythritoldiacrylat und Trimethylolpropantriacrylat im Polymerisat enthalten sein. Der Anteil dieser Comonomere an der Gesamtmonomerenmenge beträgt bis zu 10 Gew.-%, vorzugsweise 0,01 bis 2 Gew.-%.

Besonders geeignet sind Comonomere mit N-funktionellen Gruppen, darunter insbesondere (Meth)acrylamid, Allylcarbamat, Acrylnitril, N-Methylol(meth)acrylamid, N-Methylolallylcarbamat sowie die N-Methylolester, -alkylether oder Mannichbasen des N-Methylol(meth)acrylamids oder N-Methylolallylcarbamats, Acrylamidoglykolsäure, Acrylamidomethoxyessigsäuremethylester, N-(2,2-Dimethoxy-1-hydroxyethyl)acrylamid, N-Dimethylaminopropyl(meth)acrylamid, N-Methyl-(meth)acrylamid, N-Butyl(meth)acrylamid, N-Cyclohexyl(meth)acrylamid, N-Dodecyl(meth)acrylamid, N-Benzyl(meth)acrylamid, p-Hydroxyphenyl(meth)acrylamid, N-(3-Hydroxy-2,2-dimethylpropyl)methacrylamid, Ethylimidazolidonmethacrylat, N-Vinylformamid, N-Vinylacetamid, N-Methylol-N-vinylacetamid, N-Vinylpyrrolidon, N-Hydroxyethyl(meth)acrylamid, N-Hydroxypropyl(meth)acrylamid, N-Methylolmaleamid, N-Methylol-maleamsäure und deren Ester mit aliphatischen (C₁-C₁₈)-Alkoholen sowie die N-Methylolamide aromatischer Vinylcarbonsäuren wie beispielsweise N-Methylol-p-vinylbenzamid. Die N-Methylolamide der Acrylsäure und Methacrylsäure sind im Falle der Verwendung der erfindungsgemäß hergestellten Dispersionen als wasserresistente Klebstoffe besonders bevorzugt. Der Anteil dieser Comonomere an der Gesamtmonomerenmenge beträgt bis zu 10 Gew.-%, vorzugsweise 0,1 bis 5 Gew.-%.

Weiterhin geeignete Comonomere sind Hydroxy-funktionelle Monomere wie 2-Hydroxyethyl(meth)acrylat, 3-Hydroxypropyl(meth)acrylat sowie deren Addukte mit Ethylenoxid oder Propylenoxid. Der Anteil dieser Comonomeren an der Gesamtmonomerenmenge kann bis zu 10 Gew.-%, vorzugsweise 0,1 bis 5 Gew. % betragen.

Weiterhin geeignet sind Comonomere, die über Carbonylgruppen vernetzbare oder selbstvernetzende Gruppen besitzen wie Diacetonacrylamid, Allylacetoacetat, Vinylacetoacetat sowie Acetoacetoxyethyl(meth)acrylat. Der Anteil dieser Comonomere an der Gesamtmonomerenmenge beträgt bis zu 10 Gew.-%, vorzugsweise 0,01 bis 5 Gew.-%.

Der Anteil der neben den Vinylestereinheiten im Polymerisat enthaltenen Comonomereinheiten beträgt zusammengenommen bis zu 50 Gew.-%.

Stabilisiert wird die im Sinne der Erfindung hergestellte homo- oder copolymere Polyvinylesterdispersion durch Schutzkolloide, die entweder bereits in der wäßrigen Polymerisationsflotte vorgelegt werden oder anteilig vorgelegt und während der Polymerisation dosiert oder zum Teil auch nachträglich zugegeben werden können. Die verwendeten Schutzkolloide können von den im eingesetzten Hydrosol enthaltenen gleich oder verschieden sein. Wird das zuvor hergestellte Hydrosol der Zirkonsalzkomplexe komplett im Eintopfverfahren direkt als Polymerisationsflotte eingesetzt, sind die bereits in der wäßrigen Phase des Hydrosols gelösten Schutzkolloide, insbesondere Polyvinylalkohol, naturgemäß vor der Polymerisation vorgelegt. Darüber hinaus können natürlich auch bei Bedarf weitere Schutzkolloide, insbesondere Polyvinylalkohol, in Form einer wäßrigen Lösung vor der Polymerisation zugegeben, während der Polymerisationsreaktion zudosiert oder nachträglich zugegeben werden.

Vorzugsweise geeignet zur Herstellung und Stabilisierung der Polyvinylesterdispersion ist Polyvinylalkohol, insbesondere Polyvinylalkohol vom Hydrolysegrad 60 bis 100 Mol.-%, vorzugsweise 70 bis 98 Mol.-%, und Viskositäten der 4 Gew.-%igen wäßrigen Lösungen bei 20 °C von 2 bis 70 mPa·s. Weiterhin können als Schutzkolloide funktionalisierte Polyvinylalkohole eingesetzt werden. Hierunter werden Verbindungen verstanden, die entweder durch nachträgliche Umsetzung mit geeigneten Verbindungen oder Copolymerisation geeigneter Monomere über funktionelle Gruppen, wie Amino-, Carboxyl- oder Acetoacetylgruppen verfügen. Beispielhaft genannt seien Umsetzungsprodukte des Polyvinylalkohols mit Diketen oder copolymere Polyvinylalkohole mit Crotonsäure- oder Vinylamineinheiten.

Weiterhin können als Schutzkolloide veretherte Cellulosederivate, beispielsweise Hydroxyethylcellulose oder Carboxymethylcellulose, oder Polyvinylpyrrolidon, Polyacrylsäure, Copolymere aus α-Olefinen mit Maleinsäure, Maleinsäurederivaten oder Maleinsäureanhydrid, Copolymere aus Styrol und Maleinsäure, Maleinsäurederivaten oder Maleinsäureanhydrid eingesetzt werden. Diese werden vorzugweise in Kombination mit Polyvinylalkohol verwendet. Bezogen auf den Feststoffanteil, beträgt der Anteil der polymeren Schutzkolloide inklusive der bereits zur Herstellung des Zrkoniumkomplexsalz-Hydrosols verwendeten Menge an Polyvinylalkohol oder anderen polymeren Stabilisatoren vorzugsweise 1 bis 20 Gew.-%, insbesondere 2 bis 12 Gew.-%.

Außerdem können zusätzlich zu den polymeren Schutzkolloiden bis zu 3 Gew.-% einschließlich der bereits gegebenenfalls zur Herstellung des Hydrosols verwendeten Menge, bezogen auf das Polymerisat, nichtionische und/oder ionische Emulgatoren in der Polymerisationsflotte mitverwendet werden.

Ein weiterer Gegenstand der vorliegenden Erfindung ist ein Verfahren zur Herstellung der Polyvinylester-Dispersion durch kontinuierliche oder diskontinuierliche Emulsionspolymerisation wenigstens eines Vinylester-Monomeren und gegebenenfalls weiterer copolymerisationsfähiger Monomerer, in Gegenwart eines eine oberflächenaktive Substanz, vorzugsweise ein überwiegend polymeres Stabilisierungsmittel, insbesondere Polyvinylalkohol, enthaltenden Hydrosols eines Komplexsalzes von Metallen der 4. Nebengruppe des Periodensystems, insbesondere Zirkonium, mit einer organischen α-Hydroxy- und/oder α-Oxosäure.

Hierbei kommen wasserlösliche und/oder öllösliche Initiatorsysteme wie Peroxodisulfate, Azoverbindungen, Wasserstoffperoxid, organische Hydroperoxide oder Dibenzoylperoxid zum Einsatz. Diese können entweder für sich oder in Kombination mit reduzierenden Verbindungen wie Fe(ll)-Salzen, Natriumpyrosulfit, Natriumhydrogensulfit, Natriumsulfit, Natriumdithionit, Natrium-Formaldehyd-Sulfoxylat, Ascorbinsäure als Redoxkatalysatorsystem verwendet werden. Die Verwendung eines Redoxkatalysatorsystems, beispielsweise aus tert.-Butylhydroperoxid in Kombination mit Natriumformaldehydsulfoxylat ist bevorzugt.

Die Reaktionstemperatur während der Polymerisation beträgt 20 bis 100 °C, insbesondere 50 bis 90 °C, vorzugsweise 60 bis 85 °C.

Unter den verwendeten Polymerisationsverfahren sind die gängigen diskontinuierlichen oder kontinuierlichen Verfahrensweisen der radikalischen Emulsionspolymerisation zulässig. Vorzugsweise werden jedoch diskontinuierliche Verfahren wie Batch, kombinierte Batch-,/Zulauf- oder reine Monomerzulaufprozesse bevorzugt. Insbesondere wird ein reines Monomerzulaufverfahren angewandt. Dabei kann ein kleiner Teil (unter,10 Gew.-%) der Monomeren zum Anpolymerisieren in der das Hydrosol aus Zirkoniumkomplexsalzen enthaltenden Polymerisationsflotte vorgelegt werden. Der Polymerisationsverlauf wird dabei vorzugsweise so gesteuert, daß keine nennenswerte Monomerakkumulation im Reaktor auftritt.

Dieser Prozeß zeichnet sich außerdem gegenüber Saatpolymerisationen herkömmlicher Art durch seine Einfachheit aus und ist, da es sich um ein reines Zulaufverfahren handelt, aus Gründen der Verfahrenssicherheit von Vorteil.

Die Erfindung erlaubt in einfacher und verfahrenssicherer Vorgehensweise vermutlich infolge einer Unterdrückung der Polymerpartikelaggregation während der Polymerisation eine hervorragende Rheologiekontrolle der als Endprodukte erhaltenen Dispersionen.

Während der Emulsionspolymerisation werden die Hydrosol-Aggregate vermutlich durch nicht näher untersuchte Prozesse abgebaut, so daß keinerlei Störung der Eigenschaften der verfilmten Dispersion beobachtet wird.

Gegenstand der Erfindung ist auch die Verwendung der Polyvinylester-Dispersionen als Beschichtungs- oder Klebemittel, insbesondere als normale oder wasserresistente Klebstoffe für poröse oder semiporöse Substrate, beispielsweise als Holz- oder Papierklebstoff sowie zum Verkleben von Preßschichtstoffen, Faserleder, Textilien. Ihre Eignung liegt vorzugsweise in Bereichen, in denen eine hohe Scher- und Lagerstabilität der Endprodukte eine Gebrauchsanforderung darstellt.

Zur Konfektionierung der als Klebstoffe verwendeten Endprodukte können der Polyvinylester-Dispersion noch übliche Additive zugesetzt werden, beispielsweise Filmbildehilfsmittel zur Erniedrigung der Filmbildungstemperatur (MFT), beispielsweise Butyldiglykolacetat, Weichmacher, Entschäumer, Füllstoffe und Konservierungsmittel.

Werden die unter Verwendung von vernetzbaren oder selbstvernetzenden Comonomeren, beispielsweise N-Methylol(meth)acrylamid, hergestellten Dispersionen als wasserresistenter Klebstoff mit langen Topfzeiten verwendet, werden vorzugsweise noch wäßrige Lösungen von Mineralsäuren mit pKₛ-Werten von < 2,5 beispielsweise Phosphorsäure, oder saure wasserlösliche Salze, beispielsweise Aluminiumchlorid, Aluminiumnitrat oder Zirkonoxychlorid, als Vernetzungskatalysatoren zugesetzt. Danach erfüllen diese Klebstoffe mindestens die Beanspruchungsgruppe D2, vorzugsweise die Beanspruchungsgruppe D3, gemäß der Norm DIN EN 204.

Die folgenden Beispiele dienen zur Erläuterung der Erfindung. Die in den Beispielen angegebenen Teile und Prozente beziehen sich auf das Gewicht, falls nicht anders vermerkt.

### Beispiele

### Herstellung von mit Polyvinylalkohol stabilisierten Komplexsalz-Hydrosolen des Zr (IV)

### Beispiele H1 bis H7

Mit teilverseiftem Polyvinylalkohol vom Hydrolysegrad 88 Mol-% und den jeweils in der Tabelle 1 aufgeführten Viskositäten der 4%igen wäßrigen Lösungen bei 20 °C werden je 500 g wäßrige Lösungen mit den in der Tabelle 1 angegebenen Konzentrationen hergestellt. In diesen mittels eines Flügelrührers (100 U/Min.) gerührten Lösungen werden bei 50 °C je 1,56 g (10,5 mmol) Glyoxylsäure (50 %ig) gelöst. Nach 10 Minuten werden je 5,88 g (10,5 mmol Zr) einer handelsüblichen Lösung von Zirkoniumacetat (22 %ig bez. auf ZrO₂) innerhalb einer Zeitspanne von 10 Minuten zudosiert. Nach weiteren 30 Minuten, innerhalb denen die Ansätze eine milchig-trübe Konsistenz annehmen, wird auf Raumtemperatur abgekühlt.

Die Partikelgrößen der Hydrosole werden mit Hilfe der Photonenkorrelationsspektroskopie an frischen Proben bestimmt. Bei den angegebenen Werten für d_{z} handelt es sich um die Z-Mittelwerte in der Kumulanten-Auswertung der monomodalen Verteilung (Gerät: Malvern 4700, Streuwinkel 90 °C; zur Meßmethodik s. a. J. P. Fischer und E. Nölken, Progr. Colloid & Polymer Sci. 77, 180 (1988)). Die aufgeführten Bereiche grenzen die gemessenen Z-Mittelwerte der Partikelgrößen aus jeweils mindestens 2 Reproduktionsansätzen ein.

**Tabelle 1:**

| Beispiel | Visk.der 4%igen wäßrigen Lösung des PVA's [mPa·s] (M_{w} [g/mol]) | Konz. der Lösung [%] | Masse PVA/Stoffmenge Zr [g/mmol] | d_{z} [µm] (Bereich) | d_{z} [µm] (Mittelwert) |
|---|---|---|---|---|---|
| H1 | 26 | 1 | 0,5 | 2,1-3,0 | 2,5 |
| | (160.000) | | | | |
| H2 | 26 | 7 | 3,3 | 1,2-2,0 | 1,6 |
| | (160.000) | | | | |
| H3 | 26 | 13 | 6,2 | 0,8-1,4 | 1,1 |
| | (160.000) | | | | |
| H4 | 4 | 7 | 3,3 | 2,6-5,9 | 4,1 |
| | (31.000) | | | | |
| H5 | 8 | 7 | 3,3 | 2,6-4,2 | 3,6 |
| | (67.000) | | | | |
| H6 | 18 | 7 | 3,3 | 1,7-4,3 | 3,0 |
| | (130.000) | | | | |
| H7 | 40 | 7 | 3,3 | 0,9-2,0 | 1,4 |
| | (205.000) | | | | |

Anhand des Gangs in den Bereichen der Z-Mittelwerte bzw. in den aus den (hier nicht aufgeführten) Einzelwerten gebildeten Mittelwerten ist ersichtlich, daß sowohl mit steigenden Mengen des Stabilisierungsmittels Polyvinylalkohol als auch - bei Verwendung gleicher Mengen - mit Erhöhung der Viskositäten der 4 %igen Lösungen und damit der Erhöhung des Molekulargewichts der Polyvinylalkohole die Feinteiligkeit der Hydrosole zunimmt.

### Herstellung von Polyvinylacetat-Dispersionen in Gegenwart von mit Polyvinylalkohol stabilisierten Komplexsalz-Hydrosolen des Zr (IV)

### Beispiele 1 und 2 sowie Vergleichsbeispiele V1, V2 und V3

**1) Herstellung der Polymerisationsflotten**
   In einem 10l-Glasrührkesselreaktor mit Ankerrührer (110 U/Min.), der mit Dosiervorrichtungen, Rückflußkühler, Mantelheizung und -kühlung versehen ist, werden 3440 g einer 7%igen wäßrigen Lösung von teilverseiftem Polyvinylalkohol vom Hydrolysegrad 88 Mol-% und einer Viskosität der 4%igen wäßrigen Lösung von 26 mPa·s bei 20 °C vorgelegt.
   Im Falle der Beispiele 1 und 2 werden diese Lösungen analog der Vorgehensweise des Beispiels **H2** mit 10,65 g Glyoxylsäure (50 %ig) und 40,32 g Zirkoniumacetat (22 % ZrO₂) behandelt. Die pH-Werte der Hydrosole werden anschließend mit 40,7 g 10%iger NaOH von 3 auf einen Wert von 5 eingestellt. Der Ansatz wird danach im Eintopfverfahren direkt weiterverwendet.
   Im Falle der Vergleichsbeispiels V1 und V2 (herkömmliche Vorgehensweise, ohne Flottenbehandlung) wird ohne weitere Behandlung die Polyvinylalkohollösung als Polymerisationsflotte verwendet.
   Im Falle des Vergleichsbeispiels V3 (mit Acetatpufferung) wird zunächst mit 16,56 g Essigsäure ebenfalls ein pH-Wert von 3 eingestellt. Anschließend wird mit 74,3 g 10 %iger NaOH ein pH-Wert von 5 eingestellt.
**2) Emulsionspolymerisation**
   Bei einer Innentemperatur von 50 °C werden nacheinander 4 g ®Agitan 280 (Entschäumungsmittel, Münzing-Chemie), 0,24 g ®Rongalit C (Natrium-Formaldehyd-Sulfoxylat, BASF), gelöst in 33 g Wasser, und 200 g Vinylacetat (5 Teile) zur Flotte gegeben. Die Innentemperatur wird auf 60 °C angehoben und die Polymerisation nach Zugabe einer Lösung von 1,13 g ®Trigonox AW 70 (tert.-Butylhydroperoxid 70 %ig, Akzo-Chemie) in 20 g Wasser gestartet.
   Nach dem Auspolymerisieren der Vorlage (Innentemperatur 68 °C) werden innerhalb von 3 Stunden zwei bzw. drei Zuläufe, bestehend einerseits aus 3840 g (95 Teilen) Vinylacetat mit 3,5 g Trigonox AW 70 im Falle der Beispiele 1 und V1 bzw. 3760 g (93 Teile) Vinylacetat mit 3,5 g Trigonox AW 70 neben 166,7 g (2 Teile) N-Methylolacrylamid (48 %ig) in 400 g Wasser im Falle der Beispiele 2, V2 und V3 sowie andererseits 1,2 g Rongalit C in 400 g Wasser zudosiert. Die Manteltemperatur wird so gesteuert, daß die Polymerisation rückflußfrei bei einer langsam steigenden Innentemperatur von 68 °C am Start bis ca. 80 °C verläuft. Anschließend wird mit nachträglichen Zugaben von Lösungen von 0,57 g Trigonox AW 70 und 0,18 g Rongalit C in 10 g bzw. 80 g Wasser nachpolymerisiert. Nach dem Abkühlen werden 160 g Butyldiglykolacetat zur MFT-Erniedrigung langsam in die Dispersion eingerührt.

### Analyse der Dispersionen

1. Viskositäten: Brookfield RVT (7/20), 23 °C
2. Die Bestimmung der Thixotropiefläche erfolgt an einem Rotationsviskosimeter vom Typ Rheomat 115 der Firma Contraves im Scherbereich von D = 20 bis 1000 s⁻¹. Meßbedingungen: Zeitdauer der Aufwärtskurve: 1 Minute, Haltezeit bei D = 1000 s⁻¹: 5 Minuten, Zeitdauer der Abwärtskurve: 1 Minute.
3. Die Analyse der Partikelgrößenverteilungen erfolgt mittels der in der Literatur beschriebenen Methode der Xe-Laser-Aerosol-Spektroskopie (Xe-LAS): J. P. Fischer in FH Texte der Fachhochschule Aachen, Bd. 66 (1995), sowie dort zitierte Lit. (beispielsweise Kunstharz Nachrichten 28, 12 ff (1991).

**Tabelle 2:**

| **Analysedaten der Dispersionen** | | | | | |
|---|---|---|---|---|---|
| Beispiel | Comonomer | Feststoffgehalt [%] | pH-Wert | Viskosität [Pa·s] | Thixotropiefläche (MPa·s⁻¹] |
| 1 | - | 52,7 | 4,5 | 33 | 0,23 |
| V1 | - | 52,2 | 3,8 | 102 | 2,18 |
| 2 | 2 % NMA | 49,6 | 4,5 | 15 | 0,16 |
| V2 | 2 % NMA | 50,2 | 3,9 | 78 | 3,19 |
| V3 | 2 % NMA | 50,1 | 4,9 | 53 | 1,01 |

Wie aus dieser Tabelle hervorgeht, zeichnen sich die erfindungsgemäßen Beispiele 1 und 2 mit jeweils einer sehr niedrigen Thixotropiefläche durch eine außerordentlich hohe Scherstabilität und bei vergleichbaren Feststoffgehalten durch eine deutlich niedrigere Viskosität im Vergleich zu den Beispielen V1 bzw. V2 und V3 aus. Allein durch Acetatpufferung, die ebenfalls einen positiven Effekt ausübt, wie im Beispiel V3, ist dieser Effekt nicht zu bewerkstelligen. Dieses Verhalten ist in Unterschieden in den Partikelgrößenverteilungen zu begründen.

**Tabelle 3:**

| **Analyse der Partikelgrößenverteilungen** | | |
|---|---|---|
| Beispiel | Lichtmikroskopie | Xe-LAS: d_{w} (Bereich) |
| 1 | überwiegend Einzelpartikel, daneben wenig Aggregate | bimodale Verteilung 0,4 - 0,8 µm (EP) neben 1,5 - 3 µm (Agg.) |
| V1 | wenig Einzelpartikel, überwiegend Partikelaggregate | bimodale Verteilung 0,3 - 0,6 µm (EP) neben 0,9 - 3 µm (Agg.) |
| 2 | überwiegend Einzelpartikel, daneben kleinere Aggregate | bimodale Verteilung 0,4 - 0,8 µm (EP) neben 1,5 - 3 µm (Agg.) |
| V2 | überwiegend Partikelaggregate | 1,5 - 4 µm (Agg.) |
| V3 | überwiegend Partikelaggregate | 1,5 - 4 µm (Agg.) |

### Beispiele 2-A und Vergleichsbeispiele V2-A und V3-A

### Prüfung der copolymeren Polyvinylacetat-Dispersionen in Kombination mit einem sauren Härter als wasserresistenter Holzklebstoff

In je 1000 Gewichtsteile der Dispersionen der Beispiele 2, V2 und V3 rührt man 27 Gewichtsteile einer 28 %igen Lösung von Aluminiumchlorid in Wasser ein. Die Ermittlung der klebstofftechnischen Kenndaten erfolgt anhand der Prüfnorm DIN EN 204 (vormals DIN 68602). Die Herstellung der dazu erforderlichen Prüfkörper erfolgt nach der Vorgehensweise der DIN EN 205 (vormals DIN 53254). Die Verleimung und Prüfung wird unter Berücksichtigung folgender Kenndaten durchgeführt:

| | |
|---|---|
| Leimauftrag | 150 ± 20 g/m² beidseitiger Auftrag |
| Offene Wartezeit | 3 Minuten |
| Geschlossene Wartezeit | 3 Minuten |
| Preßzeit | 2 Stunden |
| Preßdruck | 0,7 ± 0,1 N/mm² |
| Anzahl Prüfkörper pro Prüffolge | 20 |

| | |
|---|---|
| Prüfung nach | |
| Lagerungsfolge gemäß DIN EN 204 D1/1 | 7 Tage Normalklima ^{*)} |
| Lagerungsfolge gemäß DIN EN 204 D3/3 | 7 Tage Normalklima |
| | 4 Tage in kaltem Wasser |
| Lagerungsfolge gemäß D1/80°C | 7 Tage Normalklima |
| (nicht Bestandteil der DIN EN 204) | 2 Stunden Wärmeschrank 80°C (Prüfung bei 80°C) |

| | |
|---|---|
| (*⁾ 23 ± 2 °C und 50 ± 5 % relative Luftfeuchte) | |

| | |
|---|---|
| Prüftemperatur | 23 °C ± 2 °C |
| Vorschubgeschwindigkeit | 50 mm/Min. |

Die Einordnung in die Beanspruchungsgruppe D1/1 erfolgt bei einer Reißfestigkeit von > 10 N/mm².
Die Einordnung in die Beanspruchungsgruppe D3/3 erfolgt bei einer Reißfestigkeit von ≥ 2 N/mm².

### Ermittlung der Topfzeit nach Härterzugabe

Im Lagerungsversuch bei Raumtemperatur wird hierunter der Zeitraum verstanden, in dem die Klebstoffdispersion nach Härterzusatz mit einer Viskosität von ≤ 75 Pa·s nach Brookfield RVT 7/20 noch sicher fließfähig ist und nach dieser Zeit die klebstofftechnischen Normwerte erfüllt.

Die erhaltenden Prüdaten sind in der nachfolgenden Tabelle 4 aufgeführt. Diese zeigen, daß sowohl das Beispiel 2-A des erfindungsgemäßen Verfahrens als auch die Vergleichsbeispiele die geforderten Normprüfwerte erfüllen. Beispiel 2-A zeichnet sich darüber hinaus durch eine drastische Verlängerung der Topfzeit aus. Nach einer Zeitspanne von 2 Monaten werden nach wie vor sämtliche Normprüfwerte erfüllt.

**Tabelle 4**

| Beispiel | 2-A | V2-A | V3-A |
|---|---|---|---|
| Visk. Brookfield RVT 7/20 Start [Pa·s] | 9 | 64 | 47 |
| D1/1 [N/mm²] | 15,8 | 15,4 | 16,9 |
| D1/80 °C [N/mm²] | 8,3 | 8,4 | 8,5 |
| D3/3 [N/mm²] | 3,5 | 4,5 | 5,1 |
| Topfzeit | >> 6 Wochen | < 14 Tage | < 14 Tage |
| D1/1 nach 2 Monaten [N/mm²] | 16,9 | - | - |
| D1/80 °C nach 2 Monaten [N/mm²] | 7,8 | - | - |
| D3/3 nach 2 Monaten [N/mm²] | 5,8 | - | - |

## Patentansprüche

1. Eine oberflächenaktive Substanz enthaltendes Hydrosol eines Komplexsalzes eines Metalls der 4. Nebengruppe des Periodensystems mit einer organischen α-Hydroxy- und/oder α-Oxosäure.

2. Hydrosol nach Anspruch 1, dadurch gekennzeichnet, daß die organische Säure eine mono- oder polyfunktionelle Carbonsäure ist, die in α-Stellung zu mindestens einer Carboxylgruppe eine Carbonylgruppe aufweist, die in wäßriger Phase im Gleichgewicht in vollständig oder partiell hydratisierter Form vorliegt.

3. Hydrosol nach Anspruch 1, dadurch gekennzeichnet, daß die organische Säure Glyoxylsäure ist.

4. Hydrosol nach Anspruch 1, dadurch gekennzeichnet, daß das Metall der 4. Nebengruppe Zirkonium ist.

5. Hydrosol nach Anspruch 1, dadurch gekennzeichnet, daß die oberflächenaktive Substanz Polyvinylalkohol ist.

6. Verfahren zur Herstellung eines Hydrosols eines Komplexsalzes eines Metalls der 4. Nebengruppe des Periodensystems mit einer organischen α-Hydroxy- und/oder α-Oxosäure nach Anspruch 1 durch Fällung aus homogener wäßriger Lösung eines wasserlöslichen Salzes des Metalls durch Zugabe einer organischen α-Hydroxy- und/oder α-Oxosäure oder einer wäßrigen oder organischen Lösung der organischen Säure in Gegenwart einer oberflächenaktiven Substanz.

7. Polyvinylester-Dispersion, erhältlich durch Emulsionspolymerisation wenigstens eines Vinylester-Monomeren und gegebenenfalls weiterer copolymerisationsfähiger Monomere in Gegenwart eines eine oberflächenaktive Substanz enthaltenden Hydrosols eines Komplexsalzes eines Metalls der 4. Nebengruppe des Periodensystems mit einer organischen α-Hydroxy- und/oder α-Oxosäure.

8. Verfahren zur Herstellung einer Polyvinylester-Dispersion durch kontinuierliche oder diskontinuierliche radikalische Emulsionspolymerisation wenigstens eines Vinylester-Monomeren und gegebenenfalls weiterer copolymerisationsfähiger Monomere in Gegenwart eines eine oberflächenaktive Substanz enthaltenden Hydrosols eines Komplexsalzes eines Metalls der 4. Nebengruppe des Periodensystems mit einer organischen α-Hydroxy- und/oder α-Oxosäure.

9. Polyvinylester-Dispersion nach Anspruch 7, dadurch gekennzeichnet, daß als Vinylestermonomer Vinylacetat eingesetzt wird.

10. Polyvinylester-Dispersion nach Anspruch 8, dadurch gekennzeichnet, daß als weiteres copolymerisationsfähiges Monomer ein selbstvernetzendes oder nachträglich vernetzbares Monomer eingesetzt wird.

11. Verwendung einer Polyvinylester-Dispersion nach Anspruch 7 als Beschichtungs- oder Klebemittel.

## Claims

1. A hydrosol of a complex salt of a metal from subgroup 4 of the Periodic Table of the Elements with an organic α-hydroxy and/or α-oxo acid, which hydrosol comprises a surface-active substance.

2. A hydrosol as claimed in claim 1, wherein the organic acid is a mono- or polyfunctional carboxylic acid which, in the α-position to at least one carboxyl group, has a carbonyl group which in the aqueous phase at equilibrium is in completely or partially hydrated form.

3. A hydrosol as claimed in claim 1, wherein the organic acid is glyoxylic acid.

4. A hydrosol as claimed in claim 1, wherein the metal from subgroup 4 is zirconium.

5. A hydrosol as claimed in claim 1, wherein the surface-active substance is polyvinyl alcohol.

6. A process for the preparation of a hydrosol of a complex salt of a metal from subgroup 4 of the Periodic Table of the Elements with an organic α-hydroxy and/or α-oxo acid as claimed in claim 1 by precipitation, from homogeneous aqueous solution, of a water-soluble salt of the metal by adding an organic α-hydroxy and/or α-oxo acid or an aqueous or organic solution of the organic acid in the presence of a surface-active substance.

7. A polyvinyl ester dispersion which is obtainable by emulsion polymerization of at least one vinyl ester monomer and, if desired, further copolymerizable monomers in the presence of a hydrosol of a complex salt of a metal from subgroup 4 of the Periodic Table of the Elements with an organic α-hydroxy and/or α-oxo acid, which hydrosol comprises a surface-active substance.

8. A process for the preparation of a polyvinyl ester dispersion by continuous or discontinuous, free-radical emulsion polymerization of at least one vinyl ester monomer and, if desired, further copolymerizable monomers in the presence of a hydrosol of a complex salt of a metal from subgroup 4 of the Periodic Table of the Elements with an organic α-hydroxy and/or α-oxo acid, which hydrosol comprises a surface-active substance.

9. A polyvinyl ester dispersion as claimed in claim 7, wherein vinyl acetate is employed as vinyl ester monomer.

10. A polyvinyl ester dispersion as claimed in claim 7, wherein an autocrosslinking or subsequently crosslinkable monomer is employed as further copolymerizable monomer.

11. The use of a polyvinyl ester dispersion as claimed in claim 7 as an adhesive or coating composition.

## Revendications

1. Substance tensioactive contenant un hydrosol d'un sel complexe d'un métal du sous-groupe 4. de la Classification périodique des éléments avec un acide organique α-hydroxy et/ou α-oxo.

2. Hydrosol selon la revendication 1, caractérisé en ce que l'acide organique est un acide carboxylique mono ou polyfonctionnel, qui présente un groupe carbonyle en position α par rapport à au moins un groupe carboxyle, qui existe en phase aqueuse à l'équilibre sous forme totalement ou partiellement hydratée.

3. Hydrosol selon la revendication 1, caractérisé en ce que l'acide organique est l'acide glyoxylique.

4. Hydrosol selon la revendication 1, caractérisé en ce que le métal est le zirconium du sous-groupe 4. de la Classification périodique.

5. Hydrosol selon la revendication 1, caractérisé en ce que la substance tensioactive est l'alcool polyvinylique.

6. Procédé de préparation d'un hydrosol d'un sel complexe d'un métal du sous-groupe 4. de la Classification périodique des éléments avec un acide organique α-hydroxy et/ou α-oxo selon la revendication 1 par précipitation d'une solution aqueuse homogène d'un sel soluble dans l'eau du métal par addition d'un acide organique α-hydroxy et/ou α-oxo ou d'une solution aqueuse ou organique de l'acide organique en présence d'une substance tensioactive.

7. Dispersion de polyester de vinyle, qu'on peut obtenir par polymérisation en émulsion d'un monomère d'ester de vinyle et éventuellement d'autres monomères copolymérisables en présence d'au moins un hydrosol d'un sel complexe d'un métal du sous-groupe 4. de la Classification périodique des éléments avec un acide organique α-hydroxy et/ou α-oxo, contenant une substance tensioactive.

8. Procédé de préparation d'une dispersion de polyester de vinyle par polymérisation radicalaire en émulsion continue ou discontinue d'au moins un monomère d'ester de vinyle et éventuellement d'autres monomères copolymérisables en présence d'au moins un hydrosol d'un sel complexe d'un métal du sous-groupe 4, de la Classification périodique des éléments avec un acide organique α-hydroxy et/ou α-oxo, contenant une substance tensioactive.

9. Dispersion de polyester de vinyle selon la revendication 7, caractérisée en ce qu'on utilise comme monomère d'ester de vinyle l'acétate de vinyle.

10. Dispersion de polyester de vinyle selon la revendication 8, caractérisée en ce qu'on utilise comme autre monomère copolymérisable un monomère autoréticulant ou réticulable ultérieurement.

11. Utilisation d'une dispersion de polyester de vinyle selon la revendication 7 comme produit de revêtement ou de collage.
